# EUROPEAN PATENT SPECIFICATION

(11) **EP 3 548 123 B1**
(45) Date of publication and mention of the grant of the patent: **10.03.2021**
(21) Application number: 17804515.9
(22) Date of filing: 21.11.2017
(51) Int. Cl.: A61M 5/32, A61M 5/00, A61M 5/24

(54) **A PREFILLED INJECTION DEVICE WITH CLEANING CHAMBER**
VORGEFÜLLTE INJEKTIONSVORRICHTUNG MIT REINIGUNGSKAMMER
DISPOSITIF D'INJECTION PRÉ-REMPLI AYANT UNE CHAMBRE DE NETTOYAGE

(30) Priority: 02.12.2016 EP 16201915
(43) Date of publication of application: 09.10.2019
(73) Proprietor: Novo Nordisk A/S, 2880 Bagsværd (DK)
(72) Inventor: PEDERSEN, Simon, Munch, 2880 Bagsværd (DK); EILERTSEN, Lars, 2880 Bagsværd (DK); ARCHILLA, Rubén, 2880 Bagsværd (DK)
(86) International application number: PCT/EP2017/079839
(87) International publication number: WO 2018/099757

(56) References cited:
- WO-A1-2015/173151
- WO-A1-2016/162284
- WO-A1-2016/173895
- FR-A1- 2 571 972
- US-A- 3 587 575

## Description

### THE TECHNICAL FIELD OF THE INVENTION:

The invention relates to a medical injection device for injecting a liquid drug and especially to a pre-filled injection device for apportioning a multiple number of individual settable doses. The invention especially relates to such pen-shaped and pre-filled injection device wherein the same needle cannula is used for multiple injections and wherein the distal tip of the needle cannula is cleaned between subsequent injections.

### DESCRIPTION OF RELATED ART:

Different examples of Injection devices for injecting doses of a liquid drug and wherein the distal tip of the needle cannula is cleaned between injections are disclosed in US 3,354,881, US 4,416,663 and WO 2015/173151 Further related art can be found in WO 2016/173895, WO 2016/162284, US 3,587,575, and FR 2,571,972.

The injection device disclosed in WO 2015/173151 has a telescopically movable needle shield which covers the distal tip of the needle cannula between injections. This movable needle shield carries a cleaning chamber which contains the same preservative containing liquid drug as present inside the cartridge. Since the liquid drug contains a preservative this preservative keeps the distal tip of needle cannula clean from various bacteria when the distal tip of the needle cannula is located inside the cleaning chamber.

The distal tip of the needle cannula is only moved outside the cleaning chamber during an injection as the telescopically movable needle shield is pushed in the proximal direction when injecting whereby the distal tip of the needle cannula penetrates through the distal septum of the cleaning chamber and into the skin of a user. When the movable needle shield is removed from the skin of the user following an injection a spring urges the shield into its initial position again covering the distal tip of the needle cannula.

The cleaning chamber is in one example filled with preservative containing liquid drug directly from the cartridge by a user operation. However, when filling preservative containing liquid drug into the cleaning chamber an overpressure can be created inside the liquid system. The cleaning chamber is therefore provided with an overflow channel that in one example terminates to the surroundings. Any surplus of liquid can thus escape through this overflow channel such that pressurising of the cleaning chamber is prevented.

However, if the preservative containing drug flows out of from the end of the overflow channel it will potentially form droplets inside the injection device. These droplets could flow to the outer surface of the injection device by flowing through some of the joints between the different parts making up the injection device. In some examples the droplets will end up being visual to the user, which could lead the user to the conclusion that the injection device is broken or have some kind of malfunction.

### DESCRIPTION OF THE INVENTION:

It is an object of the present invention to provide an injection device having an overflow channel leading to the surroundings but wherein any overflow is kept non-visual to the user at all times The invention is defined in claim 1. Advantageous embodiments are further defined in the dependent claims.

Accordingly, in a general aspect of the present invention a prefilled injection pen for apportioning multiple set doses of a preservative containing liquid drug comprises:
- a housing structure which permanently and non-removable secures a cartridge containing the preservative containing liquid drug. The cartridge or container is maintained embedded or secured in the housing structure at all times. The cartridge is both non-exchangeable and non-refillable. Once the user has emptied the cartridge or container for its injectable content the only possibility is to discharge the entire injection device as a whole,
- a needle cannula which has a distal end and a proximal end. The proximal end is connected to the cartridge to create a liquid communication and the distal end terminates in a sharp distal tip suitable to penetrate the skin of a user,
- a movable needle shield for covering at least the distal tip of the needle cannula at least between injections. The movable needle shield is movable in an axial direction in relation to the housing structure between a first position and a second position. By the term covering is meant that the needle shield, in the first position, at least visually, covers the distal tip of the needle cannula in a radial direction. The axially movable needle shield is obviously open at its distal end allowing the axially movable needle shield to move axially in the proximal direction to a second position wherein at least the distal tip of the needle cannula protrudes out through this opening, which in one embodiment could be a small hole, and
- a cleaning assembly with a chamber which is carried by the axially movable needle shield preferably at a distal end thereof such that the distal tip of the needle cannula is maintained inside the cleaning chamber between injections. The cleaning chamber contains a volume of the same preservative containing liquid drug as present in the cartridge. The cleaning chamber is further provided with an outlet which is open to the surroundings. The form or shape of this outlet can be any as long as it is possible for a quantum of the preservative containing liquid drug to escape through the outlet.

According to the invention, means are provided in the near proximity of the outlet for collecting the escaped quantum of the preservative containing liquid drug. These means can be any means which are able to prevent an overflow from flowing further out through the injection device.

Examples of such means could be:
- Any absorbing material able to absorb and hold a volume of the preservative containing liquid drug. Such absorptive material could be any kind of a porous liquid absorptive material such as a traditional sponge.
- A surface tension on a suitable surface.
- Capillary tubes or openings suitable of absorbing and retaining a liquid volume by capillary effect.
- A collection chamber able to collect a volume of the preservative containing liquid drug. Such physical collection chamber is preferably sealable in order to secure the collected volume.

No matter which kind of collecting means are provided, the means are provided in the near proximity of the outlet.

When the cleaning chamber is being filled with preservative containing liquid drug from the cartridge, the air inside the cleaning chamber will escape through the outlet. Depending on the tolerances an amount of the preservative containing liquid drug can also escape through the outlet. By providing means to collect this overflow of liquid drug it can be prevented that the preservative containing liquid drug flows through the various joints in the injection device and becomes visual to the user.

When providing the means as a plurality of capillary tubes these tubes or openings are preferably connected by radial openings allowing air inside the tubes to disappear as the drug is sucked up into the tubes. The capillary tubes can be provided in any position needed but the maximum effect is obtained if the tubes are provided near to the outlet.

In a further example, the outlet terminates into one or more grooves. As described in details in WO2017/050694 the valve mechanism comprises a hollow spacer element holding the two seals or septae in a longitudinal position spaced away from each other. The interior of the spacer element thus forms the cleaning chamber. The spacer element and the two seals or septae are further secured in an outer tube thus supporting the hollow spacer element. The hollow spacer element is in one example welded to an end element. The outer surface of the outer tube guides or least supports the valve ring such that the outer tube together with the spacer element and the valve ring are rotatable in relation to each other. The relative rotation brings the outlet into contact with a sealing material positioned on the valve ring such that the outlet is sealed by the valve ring following such relative rotation.

The outlet is in a preferred example provided in the spacer element preferably in the form of a channel or channel-like structure which extend radially from the spacer element.

This channel or channel-like structure is sealable by the valve ring which further is provided with one or more axial grooves. However, other constructions can be foreseen within the scope of the present claims. These axial grooves thus forms the means for collecting the escaped quantum of the preservative containing liquid drug.

In a further example the one or more grooves are open either at a distal end, at a proximal end or at both ends. When the one or more grooves guides the escapable quantum to the absorptive material, the absorptive material are preferably provided at the open end or ends of the one or more channels, or at least in the near proximity of the open end or ends.

In a specific example, the one or more grooves are sealed by a sealing ring such that the one or more channels which thus form a physical collection chamber can be sealed to thereby confine and encapsulate the escaped preservative containing liquid drug in order to prevent dripping from the injection device.

In order to seal the one or more channels, a sealing ring is provided which sealing ring in a relaxed state allows air and liquid to pass but which sealing ring can be compressed to thereby seal off the one or more channels.

The compression of the sealing ring is in one example done by applying an axial pressure onto the sealing ring. In one preferred example, the axial pressure is applied by moving a part of the injection device in an axial direction. The part moved is in a preferred example the valve ring or the outer tube. The sealing ring could e.g. be provided between the outer tube and the valve ring such that a relative axial movement between these two parts squeezes the sealing ring. In order to move the outer tube and the valve ring relatively to each other one of the parts can be provided with a guiding track and the other part with a protrusion engaging the guiding track. The guiding track is then further provided with a curve-shape or a raised portion such that the protrusion and the other part is moved axially when the protrusion during rotation encounters this curve or raised portion.

### DEFINITIONS:

An **"injection pen"** is typically an injection apparatus having an oblong or elongated shape somewhat like a pen for writing. Although such pens usually have a tubular cross-section, they could easily have a different cross-section such as triangular, rectangular or square or any variation around these geometries.

The term **"Needle Cannula"** is used to describe the actual conduit performing the penetration of the skin during injection. A needle cannula is usually made from a metallic material such as e.g. stainless steel and preferably connected to a needle hub to form a complete injection needle, all though the needle cannula could also be connected directly to the housing structure without a needle hub. A needle cannula could however also be made from a polymeric material or a glass material.

As used herein, the term **"drug"** is meant to encompass any drug-containing flowable medicine capable of being passed through a delivery means such as a hollow needle in a controlled manner, such as a liquid, solution, gel or fine suspension. Representative drugs includes pharmaceuticals such as peptides, proteins (e.g. insulin, insulin analogues and C-peptide), and hormones, biologically derived or active agents, hormonal and gene based agents, nutritional formulas and other substances in both solid (dispensed) or liquid form.

**"Cartridge"** is the term used to describe the container actually containing the drug. Cartridges are usually made from glass but could also be moulded from any suitable polymer. A cartridge or ampoule is preferably sealed at one end by a pierceable membrane referred to as the **"septum"** which can be pierced e.g. by the non-patient end of a needle cannula. Such septum is usually self-sealing which means that the opening created during penetration seals automatically by the inherent resiliency once the needle cannula is removed from the septum. The opposite end is typically closed by a plunger or piston made from rubber or a suitable polymer. The plunger or piston can be slidable moved inside the cartridge. The space between the pierceable membrane and the movable plunger holds the drug which is pressed out as the plunger decreased the volume of the space holding the drug. However, any kind of container - rigid or flexible - can be used to contain the drug.

Since a cartridge usually has a narrower distal neck portion into which the plunger cannot be moved not all of the liquid drug contained inside the cartridge can actually be expelled. The term **"initial quantum"** or **"substantially used"** therefore refers to the injectable content contained in the cartridge and thus not necessarily to the entire content.

**"Cleaning chamber"** is in the present description broadly meant to be any kind of reservoir containing a cleaning solvent to clean at least the distal tip of the needle cannula between subsequent injections. Such cleaning chamber is preferably both distally and proximally sealed by a pierceable septum. However, the proximal septum could be replaced by any kind of sealing which would seal against the outer surface of the needle cannula. The distal septum and the proximal septum or seal of the cleaning chamber defines a confinement containing the cleaning solvent which cleaning solvent in a preferred embodiment is identical to the preservatives contained in the liquid drug used in the specific injection device. In a most preferred solution, the same preservative containing liquid drug is present in both the cleaning chamber and in the cartridge of the injection device thereby avoiding contamination of the preservative containing drug inside the cartridge.

By the term **"Pre-filled"** injection device is meant an injection device in which the cartridge containing the liquid drug is permanently embedded in the injection device such that it cannot be removed without permanent destruction of the injection device. Once the pre-filled amount of liquid drug in the cartridge is used, the user normally discards the entire injection device. This is in opposition to a **"Durable"** injection device in which the user can himself change the cartridge containing the liquid drug whenever it is empty. Pre-filled injection devices are usually sold in packages containing more than one injection device whereas durable injection devices are usually sold one at a time. When using pre-filled injection devices an average user might require as many as 50 to 100 injection devices per year whereas when using durable injection devices one single injection device could last for several years, however, the average user would require 50 to 100 new cartridges per year.

Using the term **"Automatic"** in conjunction with injection device means that, the injection device is able to perform the injection without the user of the injection device delivering the force needed to expel the drug during dosing. The force is typically delivered - automatically - by an electric motor or by a spring drive. The spring for the spring drive is usually strained by the user during dose setting, however, such springs are usually prestrained in order to avoid problems of delivering very small doses. Alternatively, the spring can be fully preloaded by the manufacturer with a preload sufficient to empty the entire drug cartridge though a number of doses. Typically, the user activates a latch mechanism e.g. in the form of a button on, e.g. on the proximal end, of the injection device to release - fully or partially - the force accumulated in the spring when carrying out the injection.

The term **"Permanently connected"** as used in this description is intended to mean that the parts, which in this application is embodied as a cartridge and a housing assembly, requires the use of tools in order to be separated and should the parts be separated it would permanently damage at least one of the parts.

All references, including publications, patent applications, and patents, cited herein are incorporated by reference in their entirety and to the same extent as if each reference were individually and specifically indicated to be incorporated by reference and were set forth in its entirety herein.
All headings and sub-headings are used herein for convenience only and should not be constructed as limiting the invention in any way.

The use of any and all examples, or exemplary language (e.g. such as) provided herein, is intended merely to better illuminate the invention and does not pose a limitation on the scope of the invention unless otherwise claimed. No language in the specification should be construed as indicating any non-claimed element as essential to the practice of the invention. The citation and incorporation of patent documents herein is done for convenience only and does not reflect any view of the validity, patentability, and/or enforceability of such patent documents.
This invention includes all modifications and equivalents of the subject matter recited in the claims appended hereto as permitted by applicable law.

### BRIEF DESCRIPTION OF THE DRAWINGS:

The invention will be explained more fully below in connection with a preferred embodiment and with reference to the drawings in which:
- Figure 1: show a cross sectional view of the distal end of the injection device.
- Figure 2: show a close-up view of the cleaning assembly.
- Figure 3: show a perspective view of the hub.
- Figure 4: show a front-view of the hub.
- Figure 5: show the cleaning arrangement of a further embodiment.
- Figure 6: show the configuration of the guiding track.

The figures are schematic and simplified for clarity, and they just show details, which are essential to the understanding of the invention, while other details are left out. Throughout, the same reference numerals are used for identical or corresponding parts.

### DETAILED DESCRIPTION OF EMBODIMENT:

When in the following terms as "upper" and "lower", "right" and "left", "horizontal" and "vertical", "clockwise" and "counter clockwise" or similar relative expressions are used, these only refer to the appended figures and not to an actual situation of use. The shown figures are schematic representations for which reason the configuration of the different structures as well as there relative dimensions are intended to serve illustrative purposes only.

In that context it may be convenient to define that the term "distal end" in the appended figures is meant to refer to the end of the injection device which usually carries the injection needle whereas the term "proximal end" is meant to refer to the opposite end pointing away from the injection needle and usually carrying the dose dial button.

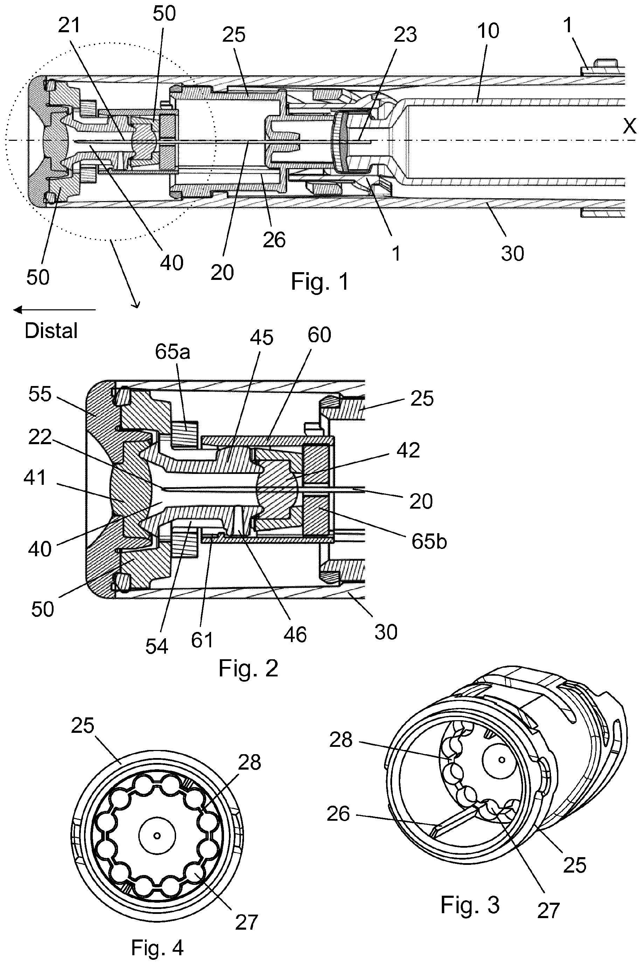

Distal and proximal are meant to be along an axial orientation extending along the longitudinal axis "X" of the injection device as further indicated in figure 1 which discloses the front end of the injection device according to a first embodiment.

The housing structure 1 secures a cartridge 10 which contains a suitable volume of a preservative containing liquid drug. As the injection device in scope is prefilled it contains only one single cartridge 10 which is permanently embedded in the housing structure 1 and which cartridge 10 is both non-refillable and non-exchangeable.

The preservative containing liquid drug contained inside the cartridge 10 is injectable through a needle cannula 20 having a distal part 21 and a proximal part 23 which proximal part 23, at least during injection, is connected to the cartridge 10, e.g. by being inserted into the cartridge 10 as disclosed in figure 1. The needle cannula 20 is preferably mounted in a hub 25, and the distal end 21 terminates in a sharp distal part 22 facilitating penetration of the skin of a user.

Between injections an axially movable needle shield 30 surrounds the needle cannula 20. This axially movable needle shield 30 is moved in the proximal direction during injection to thereby expose at least the distal part 22 of the needle cannula 20. Following an injection a not-shown spring urges the axially movable needle shield 30 back to its initial position.

The axially movable needle shield 30 further carries a cleaning assembly 15 for cleaning the distal tip 21 of the needle cannula 20 between injections. This cleaning assembly 15 which is depicted in close-up in figure 2 comprises a spacer element 45 spacing a distal seal 41 and a proximal seal 42, a surrounding valve ring 60 and an outer tube 50 locked to an end element 55.

The spacer element 45 together with the two seals 41, 42 defines a cleaning chamber 40 for cleaning the distal part 22 of the needle cannula 20 between injections as will be explained. The seals 41, 42 can be any kind of seals but are preferably two pierceable septae 41, 42 through which the needle cannula 20 is able to penetrate and which pierceable septae 41, 42 are self- sealing as it is known from a traditional septum 11 used in a cartridge 10.

Figure 1 and figure 2 discloses the distal end of the injection device wherein the axially movable needle shield 30 distally carries the cleaning assembly 15. The cleaning assembly 15 is preferably welded onto the axially movable needle shield 30 but could be connected thereto using other means such as gluing or snap fitting.

As best seen in the close-up in figure 2, the cleaning chamber 40 is sealed by a distal septum 41 and a proximal septum 42 which are separated by a spacer element 45. This spacer element 45 is together with the two septae 41, 42 provided inside an outer tube 50. Distally this outer tube 50 is welded to a distal circular end element 55. During manufacture, the two septae 41, 42 and the spacer element 45 are pressed together between the outer tube 50 and the end element 55 in the axial direction to secure that the cleaning chamber 40 is sealed in a liquid tight manner. The cleaning chamber 40 is thus defined by the inner wall of the spacer element 45 and the two septae 41, 42.

The cleaning chamber 40 is filled with preservative containing liquid drug directly from the cartridge 10 in a user initiated process. In order to make sure the cleaning chamber 40 is properly filled a radial outlet 46 is provided through which outlet 46 air and any surplus of preservative containing liquid drug filled into the cleaning chamber 40 can escape. The radial outlet 46 is preferably shaped as a channel radially terminating in an orifice.

On the outside of the outer tube 50, a valve ring 60 is provided. This valve ring 60 has one or more axial grooves 61 through which any surplus of preservative containing liquid drug can flow. The valve ring 60 which is disclosed in details in WO 2017/050694 by Novo Nordisk A/S further has a sealing material provided on the inner surface such that when the spacer element 45 and the valve ring 60 are rotated relative to each other this sealing material is brought into a position covering the radial outlet 46 thereby preventing further flow from the cleaning chamber 40.

Further, the outer tube 50 is provided with a number of longitudinal openings 54 through which the radial channel-shaped outlet 46 extend and contacts the valve ring 60. In figure 1 and 2 these longitudinal openings are seen in a cross sectional view. A perspective view is provided in figure 19 of WO 2017/050694 which is hereby incorporated by reference.

However, during filling of the cleaning chamber 40 from the cartridge 10, both the radial outlet 46 and the axial grooves 61 are open to the surroundings such that any air contained in the liquid system can escape. Due to the tolerances of the parts of the injection device it is possible that not only air but also preservative containing liquid drug will escape through both the outlet 46 and the axial grooves 61. In case the preservative containing liquid drug flows out at the open ends of the axial grooves 61 it can in some cases be seen by a user using the injection device. The user could henceforth in some cases see drops appearing on a surface of the injection device. This is highly unwanted as it could lead the user to conclude that the injection device has a defect.

To prevent this surplus of preservative containing liquid drug from becoming visual to the user an absorbing material 65a, 65b is provided at the open end of the axial grooves 61.

However, the axial channel 61 can in one example be open at both the distal end and the proximal end. As disclosed in figure 1, the absorptive material 65a, 65b can be provided at both open ends of the axial channel 61. It is also possible to provide axial grooves 61 which are only open at one end in which case the absorptive material 65a, 65b is only needed at the open end.

Figure 3 and figure 4 disclose perspective view of the hub 25 carrying the needle cannula 20. The hub 25 is on an inner surface provided with a longitudinal rib 26 which engages the valve ring 60 such that the valve ring 60 is rotated in relation to the outer tube 50 and the spacer element 45 whenever the hub 25 and the shield 30 carrying the cleaning arrangement are rotated in relation to each other. In this way the radial outlet 46 is sealed when the hub 25 and the shield 30 are rotated relatively.

In one embodiment, the hub 25 is provided with a series of capillaries channels, holes or tubes 27 into which any surplus of the preservative containing liquid drug can be drawn by the capillary effect. These capillary tubes 27 are preferably connected to each other by radial openings 28 allowing air to flow out of the capillary tubes.

In the disclosed example these capillary tubes 27 are provided in a bottom surface of the hub 25 but the capillary tubes 27 can in principle be provided in any suitable position. Further, the capillary tubes 27 can be combined with the absorptive material 65a, 65b of the previous embodiment or they can be provided as the only liquid collecting means.

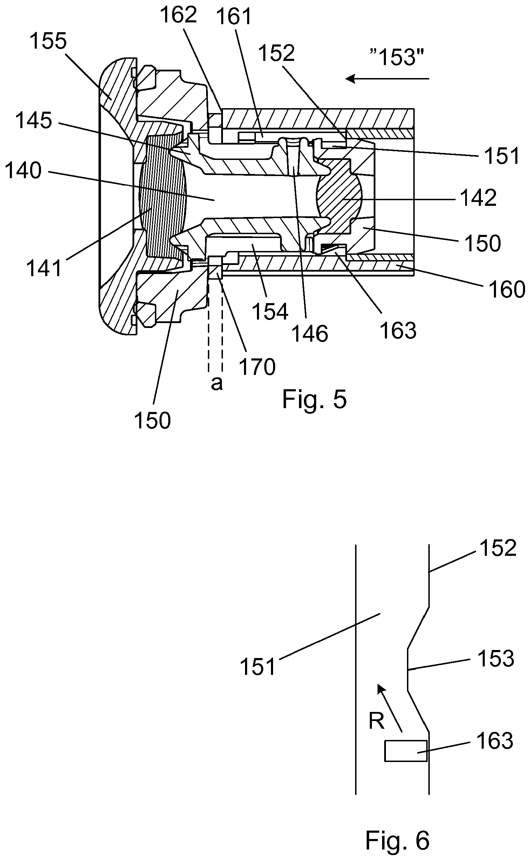

Figure 5 disclose a slightly different embodiment of the cleaning assembly 115. The same or similar elements are identified by the same reference numbers as in the first embodiment however with an "1" in front of the number. The elements of this embodiment could easily be combined with the capillary tubes 27 previously disclosed.

In this embodiment, the distal septum 141 and the proximal septum 142 are kept apart by the spacer element 145 as in the cleaning assembly in the first embodiment. The two septae 141, 142 together with the spacer element 145 are provided inside an outer tube 150 which is welded to an end element 155 to form a sealed cleaning chamber 140.

On the outside surface of the outer tube 150, a rotatable valve ring 160 is provided. This valve ring 160 is on its inner surface provided with a sealing material which can be brought into a position wherein this sealing material seals the radial outlet 146 thereby preventing further liquid to escape from the cleaning chamber 140.

The radial outlet 146 extends through longitudinal openings 154 provided in the outer tube 150. When the radial outlet 146 is not sealed it leads to one or more axial grooves 161 through which air and any surplus of preservative containing liquid drug can escape form the cleaning chamber 140.

In the axial direction the distal end surface 162 of the valve ring 160 is located and held in a position proximally to the outer tube 150 such that an air-gap (indicated by "a" in figure 5) is provided between the outer tube 150 and the valve ring 160.

In this air-gap, a sealing ring 170 is provided. The sealing ring 170 is only loosely fitted between the outer tube 150 and the valve ring 160 thereby allowing air and any surplus of preservative containing liquid drug to escape through the air-gap.

As further seen from figure 5, the valve ring 160 is in this embodiment provided with an inwardly pointing protrusion 163 which are guided in a guiding track 151 provided in the outer tube 150.

Figure 6 discloses this guiding track 151 in an unfolded state i.e. the guiding track 151 is drawn in two dimensions only. As seen the proximal sidewall 152 of this track 151 has a slightly raised portion 153 along its circular periphery.

When the valve ring 160 and the outer tube 150 rotates in relation to each other, the outwardly pointing protrusion 163 travels along the sidewall 152 of the outer tube 150. This is indicated by the arrow "R" in figure 6. When the inwardly pointing protrusion 163 engages the raised portion 153, the protrusion 163 and with it the valve ring 160 is forced to move in the distal direction a distance that equals the height of the raised portion 153. This axial movement is indicated with the arrow marked "153" in figure 5. This axial movement of the valve ring 160 applies an axial pressure onto the sealing ring 170 which thereby seals off the air-gap "a" leading to the axial channel 161.

As a result whenever the user rotates the valve ring 160 and the outer tube 150 relatively to seal off the radial outlet 146, the valve ring 170 is automatically moved axially in the distal direction thus at the same time sealing of the air-gap "a" and the axial channel 161.

Some preferred embodiments have been shown in the foregoing, but it should be stressed that the invention is not limited to these, but may be embodied in other ways within the subject matter defined in the following claims.

## Claims

1. A prefilled injection device for delivering doses of a preservative containing liquid drug comprising:
a housing structure (1) permanently securing a non-exchangeable cartridge (10) containing the preservative containing liquid drug,
a needle cannula (20) having a distal part (21) and a proximal part (23) and wherein the proximal part (23) connects to the cartridge (10) at least during dose expelling, and the distal part (21) terminates in a distal tip (22) for penetrating the skin of a user,
a movable needle shield (30) which is axially movable in relation to the housing structure (1) between a first position and a second position,
a cleaning assembly (15, 115) carried by the axially movable needle shield (30), which cleaning assembly (15, 115) comprises a cleaning chamber (40, 140) containing a volume of the same preservative containing liquid drug as present inside the cartridge (10) and which cleaning chamber (40, 140) is provided with an outlet (46, 146) through which a quantum of the preservative containing liquid drug is escapable,
wherein the distal tip (22) of the needle cannula (20) is located inside the cleaning chamber (40, 140) when the axially movable needle shield (30) is in the first position and which distal tip (22) is located distally outside the cleaning chamber (40, 140) when the axially movable needle shield (30) is in the second position, **characterized in that** the outlet (46,146) is open rroundings and **in that** means (65a, 65b, 27, 161) are provided in the near proximity of the outlet for collecting the escaped quantum of the preservative containing liquid drug.

2. A prefilled injection device according to claim 1, wherein the means for collecting the escaped quantum absorbs the escaped quantum of the preservative containing liquid drug.

3. A prefilled injection device according to claim 2, wherein the means for collecting the escaped quantum is an absorptive material (65a, 65b) such as a sponge material.

4. A prefilled injection device according to any of the claims 1 to 3, wherein the cleaning assembly (15, 115) comprises a hollow spacer element (45, 145) axially spacing a distal seal (41, 141) and a proximal seal (42, 142) to form the cleaning chamber (40, 140) there between.

5. A prefilled injection device according to claim 4, wherein the cleaning assembly (15, 115) further comprises an outer tube (50, 150) supporting the hollow spacer element (45, 145).

6. A prefilled injection device according to any of the previous claims, wherein the outlet (46, 146) is a channel.

7. A prefilled injection device according to claim 6, wherein the channel extend radially from the cleaning chamber (40, 140).

8. A prefilled injection device according to any of the claims 4 to 7, wherein the hollow spacer element (45, 145) is surrounded by a valve ring (60, 160).

9. A prefilled injection device according to claim 8, wherein the outlet (46, 146) terminates into one or more axial grooves (61, 161) provided in the valve ring.

10. A prefilled injection device according to claim 8 or 9, wherein the valve ring (60, 160) and the hollow spacer element (45, 145) are rotatable in relation to each other to thereby seal the opening (46, 146).

11. A prefilled injection device according to claim 9 or 10, wherein the one or more of the grooves (61) are open at one or both axial ends.

12. A prefilled injection device according to any of the claims 9 to 11, wherein the one or more grooves (161) are sealed by a sealing ring (170) such that the one or more grooves (161) are the means for collecting the escaped quantum of the preservative containing liquid drug.

13. A prefilled injection device according to claim 12, wherein the sealing ring (170) is axially compressible by an axial movement of the valve ring (160).

14. A prefilled injection device according to claim 13, wherein the valve ring (160) is guided in a guiding track (151) to move axially during relative rotation between the outer tube (150) and the valve ring (160).

15. A prefilled injection device according to claim 14, wherein one of the valve ring (160) or the outer tube (150) are provided with a protrusion (163) guided in the guiding track (151) which is provided in the other of the valve ring (160) or the outer tube (150).

## Patentansprüche

1. Vorbefüllte Injektionsvorrichtung zur Abgabe einer Dosis eines Konservierungsmittel enthaltenden flüssigen Arzneimittels, umfassend:
eine Gehäusestruktur (1), die eine nicht austauschbare Patrone (10), die das Konservierungsmittel enthaltende flüssige Arzneimittel enthält, dauerhaft sichert,
eine Nadelkanüle (20), die einen distalen Abschnitt (21) und einen proximalen Abschnitt (23) aufweist und wobei der proximale Abschnitt (23) zumindest während des Ausstoßens der Dosis mit der Patrone (10) verbunden ist und der distale Abschnitt (21) in einer distalen Spitze (22) zum Eindringen in die Haut eines Benutzers endet,
einen beweglichen Nadelschutz (30), der in Bezug auf die Gehäusestruktur (1) zwischen einer ersten Position und einer zweiten Position axial beweglich ist,
eine Reinigungsanordnung (15, 115), die von dem axial beweglichen Nadelschutz (30) getragen wird, wobei die Reinigungsanordnung (15, 115) eine Reinigungskammer (40, 140) umfasst, die ein Volumen desselben Konservierungsmittel enthaltenden flüssigen Arzneimittels beinhaltet, wie es in der Patrone (10) vorhanden ist, und wobei die Reinigungskammer (40, 140) mit einem Auslass (46, 146) versehen ist, durch den ein Quantum des Konservierungsmittel enthaltenden flüssigen Arzneimittels entweichen kann,
wobei sich die distale Spitze (22) der Nadelkanüle (20) innerhalb der Reinigungskammer (40, 140) befindet, wenn sich der axial bewegliche Nadelschutz (30) in der ersten Position befindet, und wobei sich die distale Spitze (22) distal außerhalb der Reinigungskammer (40, 140) befindet, wenn sich der axial bewegliche Nadelschutz (30) in der zweiten Position befindet, **dadurch gekennzeichnet, dass** der Auslass (46, 146) zur Umgebung hin offen ist und dass
Mittel (65a, 65b, 27, 161) in der Nähe des Auslasses zum Auffangen des ausgetretenen Quantums des Konservierungsmittel enthaltenden flüssigen Arzneimittels vorgesehen sind.

2. Vorgefüllte Injektionsvorrichtung nach Anspruch 1, wobei das Mittel zum Auffangen des ausgetretenen Quantums das ausgetretene Quantum des Konservierungsmittel enthaltenden flüssigen Arzneimittels absorbiert.

3. Vorgefüllte Injektionsvorrichtung nach Anspruch 2, wobei das Mittel zum Auffangen des ausgetretenen Quantums ein absorbierendes Material (65a, 65b) wie beispielsweise ein Schwamm-Material ist.

4. Vorgefüllte Injektionsvorrichtung nach einem der Ansprüche 1 bis 3, wobei die Reinigungsanordnung (15, 115) ein hohles Abstandselement (45, 145) umfasst, das eine distale Dichtung (41, 141) und eine proximale Dichtung (42, 142) axial beabstandet, um die Reinigungskammer (40, 140) dazwischen zu bilden.

5. Vorgefüllte Injektionsvorrichtung nach Anspruch 4, wobei die Reinigungsanordnung (15, 115) ferner ein Außenrohr (50, 150) umfasst, das das hohle Abstandselement (45, 145) trägt.

6. Vorgefüllte Injektionsvorrichtung nach einem der vorhergehenden Ansprüche, wobei der Auslass (46, 146) ein Kanal ist.

7. Vorgefüllte Injektionsvorrichtung nach Anspruch 6, wobei sich der Kanal radial von der Reinigungskammer (40, 140) erstreckt.

8. Vorgefüllte Injektionsvorrichtung nach einem der Ansprüche 4 bis 7, wobei das hohle Abstandselement (45, 145) von einem Ventilring (60, 160) umgeben ist.

9. Vorgefüllte Injektionsvorrichtung nach Anspruch 8, wobei der Auslass (46, 146) in einer oder mehreren axialen Nuten (61, 161) endet, die im Ventilring vorgesehen sind.

10. Vorgefüllte Injektionsvorrichtung nach Anspruch 8 oder 9, wobei der Ventilring (60, 160) und das hohle Abstandselement (45, 145) relativ zueinander drehbar sind, um dadurch die Öffnung (46, 146) abzudichten.

11. Vorgefüllte Injektionsvorrichtung nach Anspruch 9 oder 10, wobei die eine oder die mehreren der Nuten (61) an einem oder beiden axialen Enden offen sind.

12. Vorgefüllte Injektionsvorrichtung nach einem der Ansprüche 9 bis 11, wobei die eine oder die mehreren Nuten (161) durch einen Dichtungsring (170) abgedichtet sind, sodass die eine oder die mehreren Nuten (161) die Mittel zum Auffangen des ausgetretenen Quantums des Konservierungsmittel enthaltenden flüssigen Arzneimittels sind.

13. Vorgefüllte Injektionsvorrichtung nach Anspruch 12, wobei der Dichtungsring (170) durch eine axiale Bewegung des Ventilrings (160) axial komprimierbar ist.

14. Vorgefüllte Injektionsvorrichtung nach Anspruch 13, wobei der Ventilring (160) in einer Führungsschiene (151) geführt wird, um sich während der relativen Drehung zwischen dem Außenrohr (150) und dem Ventilring (160) axial zu bewegen.

15. Vorgefüllte Injektionsvorrichtung nach Anspruch 14, wobei entweder der Ventilring (160) oder das Außenrohr (150) mit einem Vorsprung (163) versehen ist, der in der Führungsschiene (151) geführt wird, die in dem jeweils anderen des Ventilrings (160) oder des Außenrohrs (150) vorgesehen ist.

## Revendications

1. Dispositif d'injection prérempli pour l'administration de doses d'un médicament liquide contenant un conservateur, comprenant :
une structure de logement (1) fixant de manière permanente une cartouche non échangeable (10) contenant un médicament liquide contenant un conservateur,
une canule d'aiguille (20) ayant une partie distale (21) et une partie proximale (23) et dans laquelle la partie proximale (23) se connecte à la cartouche (10) au moins pendant l'expulsion de la dose, et la partie distale (21) se termine en une pointe distale (22) pour la pénétration de la peau d'un utilisateur,
un dispositif de protection d'aiguille mobile (30) qui est mobile axialement par rapport à la structure de logement (1) entre une première position et une deuxième position,
un ensemble de nettoyage (15, 115) porté par le dispositif de protection d'aiguille mobile axialement (30), lequel ensemble de nettoyage (15, 115) comprend une chambre de nettoyage (40, 140) contenant un volume du même médicament liquide contenant un conservateur présent à l'intérieur de la cartouche (10) et laquelle chambre de nettoyage (40, 140) est fournie avec une sortie (46, 146) à travers laquelle un quantum du médicament liquide contenant un conservateur peut s'échapper,
dans lequel la pointe distale (22) de la canule d'aiguille (20) est située à l'intérieur de la chambre de nettoyage (40, 140) lorsque le dispositif de protection d'aiguille mobile axialement (30) est dans la première position et laquelle pointe distale (22) est située distalement à l'extérieur de la chambre de nettoyage (40, 140) lorsque le dispositif de protection d'aiguille mobile axialement (30) est dans la deuxième position, **caractérisé en ce que** la sortie (46, 146) est ouverte sur l'environnement et **en ce que**
un moyen (65a, 65b, 27, 161) est fourni à proximité immédiate de la sortie pour la collecte du quantum échappé du médicament liquide contenant un conservateur.

2. Dispositif d'injection prérempli selon la revendication 1, dans lequel le moyen pour la collecte du quantum échappé absorbe le quantum échappé du médicament liquide contenant un conservateur.

3. Dispositif d'injection prérempli selon la revendication 2, dans lequel le moyen pour la collecte du quantum échappé est un matériau absorbant (65a, 65b) tel qu'un matériau éponge.

4. Dispositif d'injection prérempli selon l'une quelconque des revendications 1 à 3, dans lequel l'ensemble de nettoyage (15, 115) comprend un élément d'espacement creux (45, 145) espaçant axialement un joint distal (41, 141) et un joint proximal (42, 142) pour former la chambre de nettoyage (40, 140) entre eux.

5. Dispositif d'injection prérempli selon la revendication 4, dans lequel l'ensemble de nettoyage (15, 115) comprend en outre un tube extérieur (50, 150) supportant l'élément d'espacement creux (45, 145).

6. Dispositif d'injection prérempli selon l'une quelconque des revendications précédentes, dans lequel la sortie (46, 146) est un canal.

7. Dispositif d'injection prérempli selon la revendication 6, dans lequel le canal s'étend radialement à partir de la chambre de nettoyage (40, 140).

8. Dispositif d'injection prérempli selon l'une quelconque des revendications 4 à 7, dans lequel l'élément d'espacement creux (45, 145) est entouré d'un anneau de valve (60, 160).

9. Dispositif d'injection prérempli selon la revendication 8, dans lequel la sortie (46, 146) se termine dans une ou plusieurs rainures axiales (61, 161) fournies dans l'anneau de valve.

10. Dispositif d'injection prérempli selon la revendication 8 ou 9, dans lequel l'anneau de valve (60, 160) et l'élément d'espacement creux (45, 145) sont rotatifs l'un par rapport à l'autre pour ainsi sceller l'ouverture (46, 146).

11. Dispositif d'injection prérempli selon la revendication 9 ou 10, dans lequel la ou les parmi les rainures (61) sont ouvertes à l'une ou aux deux extrémités axiales.

12. Dispositif d'injection prérempli selon l'une quelconque des revendications 9 à 11, dans lequel la ou les rainures (161) sont scellées par un anneau d'étanchéité (170) de telle sorte que la ou les rainures (161) sont les moyens pour la collecte du quantum échappé du médicament liquide contenant un conservateur.

13. Dispositif d'injection prérempli selon la revendication 12, dans lequel l'anneau d'étanchéité (170) est compressible axialement par un mouvement axial de l'anneau de valve (160).

14. Dispositif d'injection prérempli selon la revendication 13, dans lequel l'anneau de valve (160) est guidé dans un rail de guidage (151) pour se déplacer axialement durant la rotation relative entre le tube extérieur (150) et l'anneau de valve (160).

15. Dispositif d'injection prérempli selon la revendication 14, dans lequel l'un parmi l'anneau de valve (160) ou le tube extérieur (150) est pourvu d'une saillie (163) guidée dans le rail de guidage (151) qui est fourni dans l'autre parmi l'anneau de valve (160) ou le tube extérieur (150).
